# EUROPEAN PATENT APPLICATION

(11) **EP 2 215 963 A1**
(43) Date of publication of application: **11.08.2010**
(21) Application number: 10001226.9
(22) Date of filing: 05.02.2010
(51) Int. Cl.: A61B 3/113, A61F 9/01, A61B 3/103, A61F 9/008, A61B 3/15

(54) **Ophthalmic surgery system**

(30) Priority: 06.02.2009 DE 102009007858
(71) Applicant: Carl Zeiss Surgical GmbH, 73447 Oberkochen (DE)
(72) Inventor: Kübler, Christoph, 73447 Oberkochen (DE); Kraus, Martin, 73460 Hüttlingen (DE)
(74) Representative: Diehl & Partner

(57) **Abstract**

The invention relates to an ophthalmic surgery system (1) for viewing and measuring optical characteristics of an eye (8), with the ophthalmic surgery system (1) comprising optics (2), an electromechanic displacement mechanism (5), and a controller (3). The optics (2) comprises a first beam path (11) for viewing the eye (8), a second beam path (12) for probing the eye (8) with a probing beam, and a third beam path (13) for detecting a part of a probing beam reflected from the eye (8). The controller (3) is adapted to determine a position of a center of an iris of the eye (8) relative to an optical axis of the first beam path (11a), and to control an actuator of the electromechanic displacement mechanism (5) in order to reposition the second beam path (12) based on the determined position of the center of the iris relative to the optical axis (11a) of the first beam path (11).

## Description

### Cross-References to Related Applications

The present application claims priority of German Patent Application No. 10 2009 007 858.4, filed February 6, 2009, entitled "OPHTHALMIC SURGERY SYSTEM", the contents of which is hereby incorporated by reference in its entirety.

### Field of the Invention

The present invention relates to an ophthalmic surgery system as for instance a surgical microscope providing a measurement beam path, like for instance a wave front measurement beam path, for the measurement of optical characteristics of an eye.

### Background of the Invention

A conventional surgical microscope for ophthalmic surgery comprises a wave front sensor integrated in a beam path of the surgical microscope. Such system provides three beam paths, in particular a first beam path for light in the visible range referred to below as observation beam path and extending through a microscope optics comprising an objective lens and oculars for enabling an observer to view an object of interest, for example an eye on which cataract surgery is to be performed, by looking through the oculars. The optics further provides a second beam path, referred to below as illumination beam path, for directing a probing beam generated by a beam source onto an eye to be examined or operated upon. The part of the probing beam reflected from the measured object, in the present case the eye, is guided within a third beam path of the optics, referred to below as measurement beam path, onto a wave front sensor for analyzing wave fronts emanating from the measured object or eye to be operated upon.

To determine the optical characteristics of an eye to be operated upon, a probing light beam having a plane wave front is directed into the eye using the illumination beam path. Due to the optical characteristics of the eye's cornea, lens, and vitreous humor, the probing light beam is focused onto a spot on the retina of the eye. A part of the probing light beam is reflected by the retina upon being focused onto and is collimated into a beam upon passing through the optical system of the eye. Parts of the optical system of the eye can be removed during eye surgery or replaced by other components like e.g. a liquid or intraocular lens introduced into the capsule of the eye lens or into one of the eye's anterior chamber.

In case of the eye's optical system being entirely free of defects, the probing beam is focused onto a dot-shaped little spot on the retina. Vice versa, the optics of the eye reshapes the part of the probing beam reflected by the retina in this case into a parallel measurement beam having plane wave fronts. Aberrations from an ideal and defect free eye optics result in deformations of the wave fronts, which can be determined using the wave front sensor and which enable the identification of a defective vision or aberration of the optical system in the measured eye. The reliability with which the optical characteristics of an eye can be determined is directly related to the quality of the wave front measurement.

### Summary of the Invention

It is therefore desirable to provide an ophthalmic surgery system enabling improved wave front measurements of an eye.

Embodiments of a corresponding ophthalmic surgery system are adapted to determine a position of an iris of an eye under examination with respect to the optical axis, i.e. with respect to the central beam of the observation beam path of the ophthalmic surgery system, in order to reposition the illumination beam path of the wave front measuring system based on the relative position of the iris as determined.

A respective ophthalmic surgery system automatically aligns the illumination beam path of the wave front measuring system with the optical axis of an eye.

Conventional ophthalmic surgery systems require a user of the system to align the illumination beam path to the eye under examination either manually or manually controlled. In the event of the illumination beam path not passing an eye's optical axis centrally, the wave front measurement yields an incorrect determination of the eye's optical characteristics. Since a user is frequently not able to manually align the central axis of the illumination beam path to the optical axis of the measured eye with the required preciseness resulting in the determination of the eye's optical characteristics being incorrect, the vision achieved with an eye surgery is on a number of occasions below expectations.

Automatic alignment prevents an improper determination of a measured eye's optical characteristics, since it determines the current position of the eye's optical axis via the position of its iris and aligns the observation beam path accordingly.

According to an embodiment, the ophthalmic surgery system comprises three beam paths, a first beam path adapted for viewing the eye, a second beam path for directing the probing beam onto the eye, and a third beam path for guiding the part of the probing beam reflected from the eye onto a wave front sensor.

The ophthalmic surgery system of a preferred embodiment comprises a controller adapted to determine the distance between the center of the iris and the optical axis of the first beam path representing the illumination beam path and to control an actuator of an electromechanical displacement mechanism to reposition the optical axis of the second beam path based on the distance determined. The actuator of the electromechanical displacement mechanism is hereby configured to induce a mechanical movement based on an electric signal provided by the controller.

Embodiments of the ophthalmic surgery system comprise a controller including an image processing module adapted to perform pattern recognition for identifying an iris of an measured eye. It is noted that the term "module" as used in the context of this document describes a technical object that can be implemented as separate device as well as part of a more complex device or system. Image processing is preferably performed based on images of the eye generated by a camera located in the observation beam path of the ophthalmic surgery system.

According to embodiments of the ophthalmic surgery system the image processing module is adapted to determine the parameters of an ellipse embodying the form and position of an edge of the iris in an image of the eye. The image processing module of further embodiments is adapted to determine the parameters of two ellipses, with one of the ellipses embodying the outer edge of the iris and the other ellipse the inner edge of the iris. The image processing module is further adapted to determine a point of intersection of the ellipse's principal axes or of the principal axes of both ellipses, and to determine the position of the center of the iris with respect to the optical axis of the first beam path using the position of the point of intersection or the positions of both points of intersection of the ellipses' principal axes in the image of the eye generated with the camera.

The electromechanic displacement mechanism of embodiments of the ophthalmic surgery system comprises two linear guides, each equipped with an actuator for enabling a displacement of the second beam path along two axes being arranged at an angle of more than zero degrees, and preferably at an angle of ninety degrees, with respect to each other, and being aligned transverse with respect to that part of the central beam of the second beam path, which is directed into the eye during a wave front measurement. The electromechanic displacement mechanism of further embodiments additionally comprises a tilting device adapted to tilt the central beam of the second beam path.

According to embodiments of the ophthalmic surgery system, at least the central beams of the first and the second beam path are arranged coaxially to each other in a region next to the object plane of the first beam path. The sensor located in the third beam path for converting a probing beam reflected from the eye into measuring signals may be formed by a Hartmann-Shack wave front sensor, whereby the controller is adapted to determine optical characteristics of the eye from the measuring signals generated by the wave front sensor. Further embodiments of the ophthalmic surgery system comprise a display device for displaying information being reflected into the ocular beam path of the first beam path.

### Brief Description of the Drawings

The forgoing as well as other advantageous features of the invention will be more apparent from the following detailed description of exemplary embodiments of the invention with reference to the accompanying drawings. It is noted that not all possible embodiments of the present invention necessarily exhibit each and every, or any, of the advantages identified herein. In the following description of certain embodiments, reference is made to the enclosed drawings, in which
- Figure 1: shows a schematic representation of the structure of an ophthalmic surgery system having a wave front measuring system, the illumination beam path of which is aligned to the iris of an eye by automatic control,
- Figure 2: illustrates the alignment of the ophthalmic surgery system's optical axis to the optical axis of an eye under examination in an ocular view,
- Figure 3: shows a flow chart with the principal control steps for aligning the illumination beam path to the iris of an eye, and
- Figure 4: shows a flow chart illustrating a method for determining the position of the iris.

### Detailed Description of Exemplary Embodiments

In the exemplary embodiments described below, components that are alike in function and structure are designated as far as possible by alike reference numerals. Therefore, to understand the features of the individual components of a specific embodiment, the descriptions of other embodiments and of the summary of the invention should be referred to.

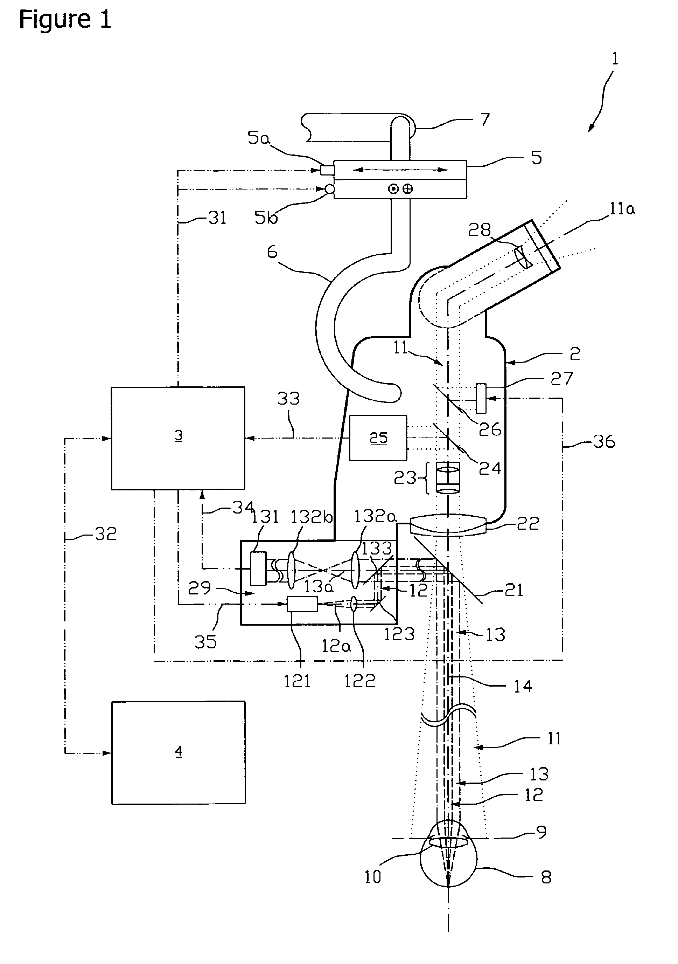

Figure 1 illustrates an ophthalmic surgery system 1 for viewing an eye 8 to be examined or operated upon in a strongly diagramatic representation. The functions of system 1 are explained below.

The ophthalmic surgery system 1 comprises an optical apparatus 2 with a first beam path 11 for viewing objects 8 arranged in the object plane 9 of the observation beam path 11, a second beam path 12 for directing a probing beam onto an object 8 to be measured, and a third beam path 13 for guiding the part of the beam path reflected from the object 8 to be measured to a sensor device. In order to avoid any confusion, the edges of each beam path are represented by a different type of lines, with the observation beam path 11 being symbolized by dotted lines, the illumination beam path 12 by dashed lines, and the measurement beam path 13 by lines where two dashes follow a single dot. The central beams and optical axes of each beam path are each symbolized by a dotdashed line 11a, 12a, and 13a.

The ophthalmic surgery system 1 further comprises a support, which in the embodiment shown comprises a fixed beam 7, an electromechanical displacement mechanism 5, and a suspension 6. Beam 7 forms the last part of a stand attached to the floor, a wall or the ceiling of a room not shown in the Figure. The optical apparatus 2, which is below also referred to as optics 2, is fixed to the suspension 6 and can be displaced relative to beam 7 by use of the electromechanic displacement mechanism 5. For effecting a respective displacement, the electromechanical displacement mechanism 5 comprises an electric actuator with two actuators 5a and 5b with just one for each of the two displacement axes. The displacement axes are preferably arranged at an angle to each other, whereby in the embodiment illustrated in Figure 1 each of the two axes is arranged transverse to the optical axis of the optical apparatus 2. The electromechanic displacement mechanism 5 preferably comprises an X-Y-table formed with two linear guides having their displacement axes arranged transverse to each other. It is noted that the term "optical axis" mentioned above is to be understood as that part of the optical axis 11a of the first beam path 11, which extends from the coupling mirror 21 onto the object 8 to be measured.

The ophthalmic surgery system 1 further comprises a controller 3 for acquiring and if necessary analyzing signals generated by the optoelectric or optoelectronic sensors arranged in the beam paths of optics 2. Controller 3 is further adapted to generate signals that are transmitted to electrooptical converters arranged in the beam paths for displaying optical information, to actuators of the ophthalmic surgery system 1 like e.g. to a switching device for controlling a light source, or to a motor for readjusting a zoom lens. Also the control of the electromechanic displacement mechanism 5 is effected using signals generated by controller 3 and transmitted to the actuators of the displacement module 5 via the transmission channel 31. The ophthalmic surgery system further comprises a user input/output module 4, enabling a user of the system to make inputs to controller 3 and enabling the controller to output information to a user. The user input/output module 4 usually comprises a keyboard and position sensitive input devices, a display device and/or a printer. Some embodiments may have the user input/output module 4 implemented in the form of a separate computer system. The communication between the user input/output module 4 and controller 3 is effected using transmission channel 32.

The transmission channels indicated in Figure 1 by lines having two dots followed by a single dash and used for transmitting signals between the controller 3 and the components communicating with it, can be implemented depending on the type of transmission used with wired lines or by radio communication. Mode and kind of the transmitted signals hereby conform to the requirements of each component controlled and the transmission channel used, whereby the signals may also represent data.

A microscope optics for viewing a part of an eye 8 located in the microscope optics' object plane 9 at a magnified scale is located in the observation path 11 of the optical apparatus 2. The microscope optics comprises an objective lens 22 formed by one or more individual lenses, and oculars 28 located, with respect to the beam path, behind the objective lens 22, i.e. on the side of the objective lens facing away from the object 8 to be measured, which enable a user to see with both eyes a magnified image of the object plane 9. Zoom lenses 23 may be arranged between object lens 22 and oculars 28 for varying an image magnification.

A partially transmitting mirror 24 may further be provided in the beam paths behind the objective lens 22 for coupling out part of the beam path's light directed to the oculars 28 and to redirect it onto a camera 25. The camera 25 generates signals representing the detected light intensities, whereby the signals are transmitted to controller 3 via transmission channel 33. Controller 3 converts the received signals or data to image data adapted for being stored or for being displayed on a display (not shown in Figure 1). The display may comprise one or more display devices or may be limited to parts of display areas of display devices, so-called windows. The display may further comprise a head-mounted-display, which can e.g. be fixed onto the head of a user with a strap.

A further partially transmitting mirror 26 for reflecting images displayed on the display device 27 into the beam path to the oculars 28 may in addition be provided in the beam paths behind the object lens 22. In the embodiment shown, the display device is controlled by controller 3 via transmission channel 36 for displaying information relevant to the user like for instance preoperative data or measurement results obtained with the wave front measurement system 29 explained further below.

The optics 2 of the ophthalmic surgery system further comprises a wave front measurement system 29 for measuring the optical characteristics of an eye 8, for instance the aberration of lens 10 or the optical system of the eye formed by the lens 10 together with the cornea and as the case may be also the vitreous humor.

The wave front measurement system 29 hereto comprises a probing beam source 121 controlled by controller 3 via the transmission channel 35, whereby the probing beam source 121 may be for example a laser diode, and emits a probing beam into the illumination beam path 12. A collimator 122 collimates the probing beam into a beam that is in the illustrated embodiment coaxially transferred into the third beam path 13 by mirrors 123 and 133, and together with this into the first beam path 11 by a further mirror 21. The collimated illumination beam directed onto the eye 8 corresponds to a substantially parallel beam with plane wave fronts. In the embodiment shown in Figure 1, the optical axes 11, 12, and 13 have the same position after being merged by mirror 21, i.e. the merged parts of the three beam paths have a common axis of symmetry 14, which is referred to as optical axis of optics 2.

The collimated illumination beam 12 is focused by the optical system of the eye onto a spot on its retina. The part of the illumination beam reflected from the spot has, at a first approximation, a cone-shaped form and is collimated by the optical system of the eye 8 into, in the ideal situation, a parallel beam with plane wave fronts. Due to usual deviations from the ideal situation like a defective vision or aberration, the probing beam reflected from the eye 8 usually shows deformed wave fronts, which are identified with the wave front measurement system 29. The reflected beam is directed to the wave front measurement system 29 via the third beam path 13. The reflected probing beam passes, after the separation of the third beam path 13 from the second beam path 12, through two lenses 132a and 132b, each of which may comprise one or more individual lenses, having the configuration of a Keppler telescope so as to properly reshape the probing beam with respect to collimation and beam cross-section. Besides the two lenses 132a and 132b also further lenses may be included in the part of the third beam path isolated from the other beam paths for reshaping the probing beam reflected from the eye 8. Instead of forming a Keppler telescope, lenses 132a and 132b may also form different optics like e.g. a Gallilei telescope.

The wave front sensor 131 of the embodiment schematically represented in Figure 1 is a Hartmann-Shack sensor comprising a (not shown) array of micro-lenses for generating an array of focal points on the two-dimensional position sensitive detector. The output signals generated by the position sensitive sensor represent the intensity distribution of the beam incident on the detector. The output signals are fed to the controller 3 via transmission channel 34, e.g. in the form of data signals. Controller 3 processes the detector signals for determining the actual position of the focal points on the detector area, and calculates the deviation of the wave fronts of the probing beam emanating from eye 8 from plane wave fronts. From this it is in turn possible to infer a presence of a defective vision or aberration of the eye's 8 optical system.

The user of the ophthalmic surgery system 1 typically initiates the measurement of the optical characteristics of an eye 8. For determining the optical characteristics of the eye 8 reliably from the shape of the wave fronts of the probing beam reflected from the eye, the illumination beam has to be precisely aligned to the optical axis of the eye 8. To put it another way, the illumination beam has to pass the pupil and thus the iris of the eye 8 centrally. In the embodiment shown in Figure 1, the three beam paths 11, 12, and 13 are merged below the mirror 21 to have a common optical axis, the optical axis of the optics 2, in this region. A respective arrangement is not mandatory, however, it enables a simple configuration of an optically controlled automatic alignment of the illumination beam to the optical axis of the eye 8.

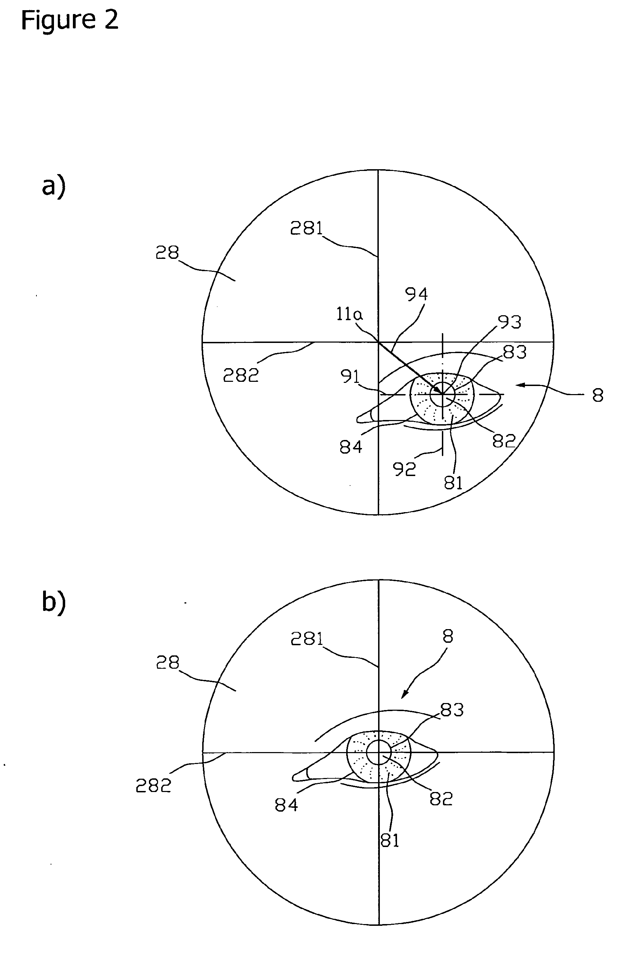

Figure 2 shows schematic representations of an eye 8 under examination as seen through oculars 28, whereby the optical axis of the eye 8 is in representation a) offset to the optical axis 11a of the optical apparatus 2, and in representation b) aligned with the optical axis 11a of the optical apparatus 2. The optical axis of the eye 8 is located at the intersection of line 91 with line 92.

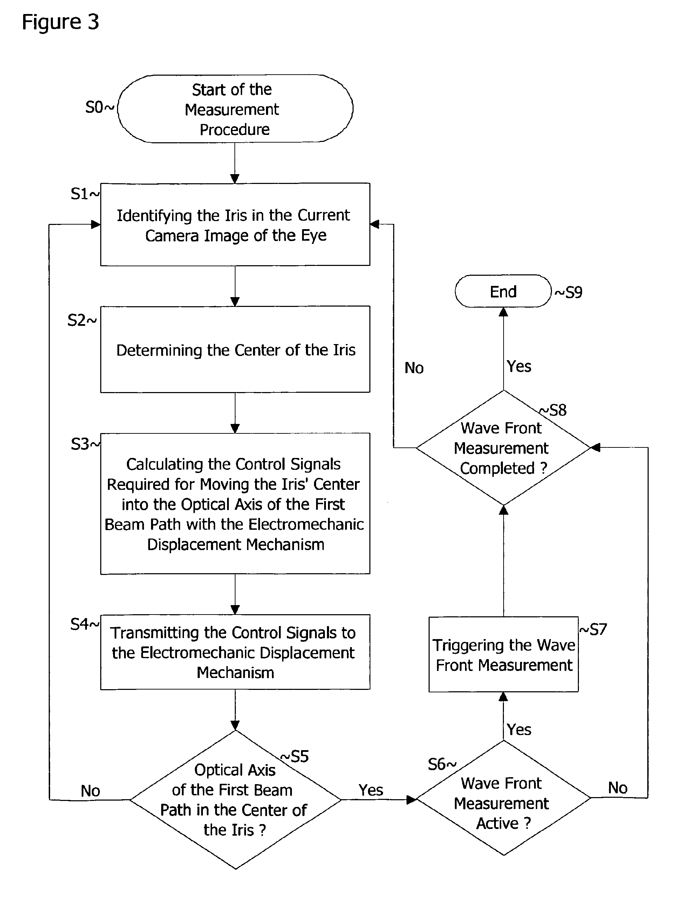

Figure 3 shows a flow chart with the basic control steps executed by the controller 3 for having the illumination beam centrally incident through the pupil of the eye 8.

After having a user of the ophthalmic surgery system 1 initiate the procedure of measuring the optical characteristics of the eye 8 to be examined in step S0, for instance by entering a respective command using the user input/output module 4, the controller 3 identifies in step S1 the image of the eye's 8 iris 81 in a current picture obtained with camera 25. The identification is accomplished by an image processing module included in the controller 8 using pattern recognition. The image processing module is preferably implemented in the form of or as part of a programmable data processing device.

In the following step S2, the controller 3 determines the center of the iris 81 in the image of the eye 8 obtained by the camera 25. The center of the iris corresponds to the position of the optical axis of the eye 8 on the surface of the eye or in the object plane 9 of optics 2.

Representation a) of Figure 2 illustrates a situation, wherein the optical apparatus 2 is positioned with its optical axis 11a offset to the optical axis of the eye 8. The offset is indicated by arrow 94. From this offset, i.e. from the position of the center of the iris 81 relative to the position of the optical axis 11a of the first beam path 11, the controller 3 now determines the signals to be transmitted to the electromechanic displacement mechanism 5 for aligning the center of the iris onto the optical axis of the first beam path 11. The transmission of the control signals to the electromechanic displacement mechanism and thus the displacement of optical apparatus is finally effected in step S4.

Several safety mechanisms can hereby be integrated for preventing that a user observing the procedure through the oculars 28 may be hurt. The displacement can for instance be carried out so slowly that the user will not be hit on his eye, or the displacement velocity may be very slow initially and then increase. Furthermore, the observer can be warned from an imminent displacement of optics 2 by acoustic signals or by information reflected from display 27 into the ocular beam path.

The above described moving of the optical axis of optics 2 to the center of the iris can be performed in one continuous operation. To account for external influences, such as a user touching or operating the optical apparatus 2, or an insufficient steadying of the patient, the centering procedure is conveniently loop controlled. A control loop like that illustrated in Figure 2 verifies in step S5, if the optical axis of the first beam path 11 is aligned to the center of the iris. This query can take place after completing the alignment, but also after only a part of the alignment has been effected. In the former case, the outcome of the center alignment is verified and adjustments are made if required. In the latter case, a controlled movement of the optical axis to the iris center is accomplished.

The alignment is completed, when the optical axis of the eye 8 is located on the optical axis 11a of the optical apparatus 2 as shown in representation b) of Figure 2. In Figure 2, the optical axis 11a of the optical apparatus is located at the intersection of the crosshair lines 281 and 282. After completion of the center alignment, it is verified in step S6, if the wave front measurement is already active. Should this not be the case, the wave front measurement is activated in step S7, i.e. initiated by the controller 3. If it has been known from step 6 that the wave front measurement has already been active at the time of the query, it is, like after an initiation of the measurement in step S7, queried in step S8, if the wave front measurement has already been completed. If this is not the case, the measurement is continued, whereby the center alignment can be repeated, according to an advantageous embodiment of the ophthalmic surgery system, beginning in step 1 for guaranteeing that the optical axis of the first beam path is not shifted from the center of the iris. If the controller 3 determines however in step S8 that the wave front measurement has been completed, the method is brought to an end in step S9.

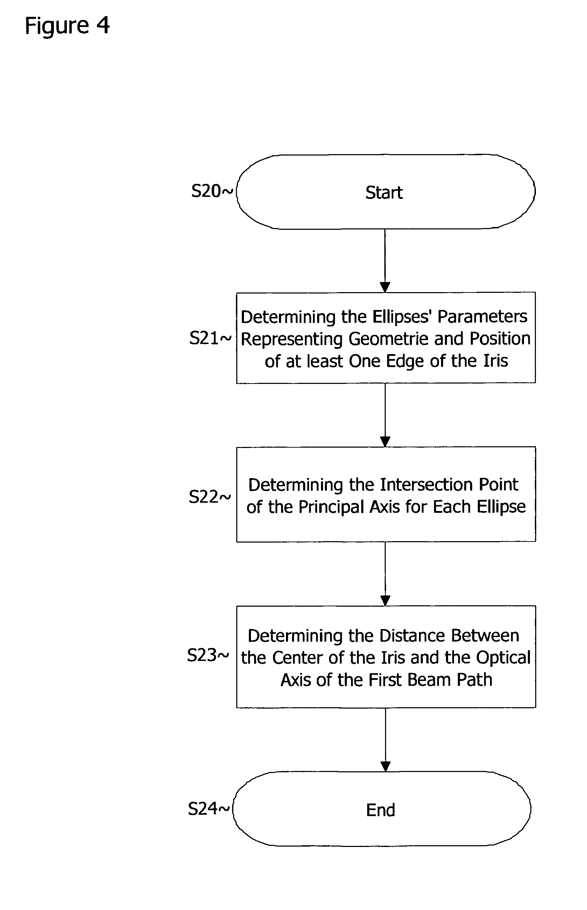

Figure 4 shows the basic steps of a method performed by the controller 3 for determining the center of the iris 81. After starting the method in step S20, which is triggered by performing step S2 of the method shown in Figure 3, the parameters of an ellipse representing the geometry and position of an edge 83 or 84 of the iris 81 identified in the camera image of the eye 8 in step S1 are determined in step S21. In a further embodiment each of the iris edges, i.e. the inner edge 83 and the outer edge 84, are represented by parameters of a corresponding ellipse. To enable an optical verification, the ellipse or ellipses can be reflected into the ocular beam path using the display device 27.

In subsequent step S22, the intersection point of the two principal axes 91 and 92 of the one or, if the parameters have been determined for both ellipses, also for the other ellipse are determined. In an ideal situation, the position of the intersection point of the principal axes of the ellipse representing the outer edge 84 of the iris coincides with that of the principal axes of the ellipse representing the inner edge 83 of the iris. When the positions of both intersection points do not coincide, both positions can be averaged for determining the center position, whereby the individual intersection point positions can be weighted with certain ellipse parameters like e.g. its linear eccentricity. It is noted that the orientation of the principal axes of the ellipses results from the actual geometry of an iris 81 and does not have to be aligned in parallel with the ocular beam path's crosshairs as shown in Figure 2.

After determining the position of the iris center and thus the position of the optical axis of the eye 8 under examination in the camera image of the eye, controller 3 determines in step S23 the distance 94 between the center of the pupil 82 or iris 81, respectively, and the position of the optical axis 11a of the first beam path 11. The camera is usually mounted immovably in the beam path, resulting in the position of the optical axis of the first beam path 11 to be known in the images obtained from the camera. The respective position is stored in a memory accessible by the controller 3. The position of the optical axis of the first beam path 11 can alternatively also be derived from the current image obtained by the camera, for example from vignetting effects or by overlaid marks. The controller 3 determines the distance between the optical axis of the eye and the optical axis 11a of the first beam path 11 directly from the displacement of the iris' center in the camera image with regards to the magnification used for the camera image. After the determination of the distance, the position of the center of the iris is known and the method is brought to an end in step 24 thereby resuming the method shown in Figure 2 with step S3.

Further advantageous embodiments of an automatic, i.e. self-centering, ophthalmic surgery system 1 as described above have an illumination beam path 12 with an optical axis 12a which does not coincide with the optical axis 11a of the first beam path 11 upon being incident on the eye 8. Different from the explained above with respect to Figures 1, 2, and 3, the center alignment is accomplished in this case by moving the central axis 12a of the illumination path 12 into the center of the iris of the eye 8 under examination instead of the central axis of the first beam path 11 of optics 2. For an optical apparatus 2 having the first beam path 11 and second beam path 12 in a fixed geometrical arrangement, the displacement of both beam paths in the object plane 9 is known and can be accounted for in steps S2, S3, and S5 of the above-described method. Besides the displacement of the two beam paths also angular deviations can be accounted for, enabling the probing beam entering the eye 8 coaxially with the eye's optical axis, while viewing of the eye under an angle to it is still possible. To this respect, the electromechanic displacement mechanism 5 further comprises a tilting degree of freedom in addition to the two linear displacement degrees of freedom, thus enabling a tilting of the optical apparatus 2.

In a further advantageous embodiment, the electromechanic displacement mechanism 5 allows a displacement and if necessary tilting of the observation beam path 12 independent of the first beam path. The optical apparatus comprises in that case dedicated sensors for determining the position of the first beam path 11 relative to the position of the second beam path 12 thus enabling a direct determination of each current position of the optical axis of the second beam path relative to the center of the iris based on the position of the iris' center relative to the optical axis 11a of the first beam path, followed by a realignment if required.

While the invention has been described with respect to certain exemplary embodiments thereof, it is evident that many alternatives, modifications and variations will be apparent to those skilled in the art. Accordingly, the exemplary embodiments of the invention set forth herein are intended to be illustrative and not limiting in any way. Various changes may be made without departing from the spirit and scope of the present invention as defined in the following claims.

## Claims

1. An ophthalmic surgery system for viewing and measuring optical characteristics of an eye (8), with the ophthalmic surgery system (1) comprising optics (2), an electromechanic displacement mechanism (5), and a controller (3),
wherein the optics (2) comprises a first beam path (11) for viewing the eye (8), a second beam path (12) for probing the eye (8) with a probing beam, and a third beam path (13) for detecting a part of a probing beam reflected from the eye (8),
wherein the electromechanic displacement mechanism (5) is adapted to displace the optical axis of the second beam path (12a) and comprises an actuator, and
wherein the controller (3) is adapted to determine a position of a center of an iris of the eye (8) relative to an optical axis (11a) of the first beam path (11) and to control the actuator of the electromechanic displacement mechanism in order to reposition the second beam path (12) based on the determined position of the center of the iris relative to the optical axis (11a) of the first beam path (11).

2. The ophthalmic surgery system according to claim 1,
wherein the controller comprises an image processing module configured to perform a pattern recognition for identifying the iris of the eye (8) in an image of the eye, wherein the image of the eye is obtained by a camera (25) located in the first beam path (11).

3. The ophthalmic surgery system according to claim 2,
wherein the image processing module is further adapted to determine an intersection point of principal axes of an ellipse representing an outline of an inner edge of the iris.

4. The ophthalmic surgery system according to claim 2 or 3, wherein the image processing module is further adapted to determine an intersection point of principal axes of an ellipse representing an outline of an outer edge of the iris.

5. The ophthalmic surgery system according to claim 2,
wherein the controller (3) is adapted to determine an intersection point of principal axes of one or more ellipses, each representing an edge of the iris, and to determine the position of the center of the iris relative to the optical axis of the first beam path (11) based on the position of the intersection point or intersection points of the principal axes in the image of the eye (8) obtained by the camera.

6. The ophthalmic surgery system according to claim 1,
wherein the electromechanic displacement mechanism (5) comprises two linear guides with the displacement axis of each being arranged transverse to the optical axis (11a) of the optical apparatus (2).

7. The ophthalmic surgery system according to claim 6,
wherein the electromechanic displacement mechanism (5) comprises an electrically operable tilting device adapted to tilt the optical axis (11a) of the optical apparatus (2).

8. The ophthalmic surgery system according to one of the preceding claims, wherein the central beams (11a, 12a) of at least the first and the second beam path are arranged coaxially to each other in a region next to the object plane (9) of the first beam path (11).

9. The ophthalmic surgery system according to one of the preceding claims, wherein the third beam path comprises a wave front sensor (131) for converting a part of a probing beam reflected from the eye (8) into measuring signals.

10. The ophthalmic surgery system according to claim 9,
wherein the wave front sensor (131) is a Hartmann-Shack type sensor.

11. The ophthalmic surgery system according to one of claims 9 or 10, wherein the controller (3) is adapted to determine optical characteristics of the eye (8) from the measuring signals generated by the wave front sensor (131).

12. The ophthalmic surgery system according to one of the preceding claims, further comprising a display device (27) for displaying information being reflected into the ocular beam path of the first beam path (11).
